# EUROPEAN PATENT SPECIFICATION

(11) **EP 1 854 497 B1**
(45) Date of publication and mention of the grant of the patent: **24.10.2012**
(21) Application number: 06713415.5
(22) Date of filing: 09.02.2006
(51) Int. Cl.: A61M 25/00

(54) **BALLOON DILATOR**
BALLONDILATATOR
DILATATEUR A BALLON

(30) Priority: 04.03.2005 JP 2005060540
(43) Date of publication of application: 14.11.2007
(73) Proprietor: OLYMPUS MEDICAL SYSTEMS CORP., Tokyo 151-0072 (JP)
(72) Inventor: SAITO, Koichiro, Tokyo (JP)
(74) Representative: von Hellfeld, Axel
(86) International application number: PCT/JP2006/302272
(87) International publication number: WO 2006/095527

(56) References cited:
- WO-A1-01/12255
- WO-A1-97/36632
- JP-A- 10 179 752
- JP-A- 2001 518 369
- JP-A- 2003 507 096

## Description

### TECHNICAL FIELD

The present invention relates to a balloon dilator used in dilation treatment applied to a narrowed or blocked part in a luminal organ of a living body.

Priority is claimed on Japanese Patent Application No. 2005-060540, filed March 4, 2005.

### BACKGROUND ART

A balloon dilator is used for dilating a narrowed or blocked part in a lumen of a living body, for example, in an operation using an endoscope for dilating a narrowed or blocked part in an alimentary canal, caused by a tumor or anastomosis performed after removal thereof

When disposing such a balloon dilator in a narrowed part of a lumen in a living body so as to dilate the narrowed part, the balloon functioning as an enlarged part may slip at a narrowed or blocked part during the dilation, and may not be positioned at a target spot for dilation. In this case, the operator must deflate the balloon, and perform re-positioning, which is a complex procedure.

Therefore, a technique has been proposed in which an uneven part is provided on the surface of the balloon, so as to prevent the balloon from slipping out from a narrowed or blocked part during inflation of the balloon (see, for example, Patent Document 1).

However, in this conventional balloon dilator, the balloon may not obtain a sufficient frictional force depending on the inner-surface state of the relevant lumen, and thus it does not provide a reliable measure for preventing slippage.

In addition, a rib or the like may be provided at the balloon, so as to change the form of a part which contacts a lumen of a living body. However, it is difficult to fabricate a balloon having such a form, and additionally, a part other than a narrowed part may be dilated in accordance with the inflation of the balloon.

Patent Document 1: Japanese Unexamined Patent Application, First Publication No. 2002-113107

WO 2001/012255 discloses a dilation balloon having a plurality of sections having different diameters when the balloon is inflated. A series of progressively larger balloon sections are used to safely dilate a stricture in stages. Each section includes a central portion having a waist for centring the balloon over the stricture, with the distal section having the smallest diameter. A wire guide extends throughout the length of the catheter.

WO 1997/036662 discloses a catheter comprising three sections, the first and third sections having balloons operably connected thereto, and the second section located between the first and third sections being more elastic than the first and third sections. In one embodiment, a pear-shaped balloon has a proximal part having a thicker wall than a distal part. The distal portion, which is wider than the proximal portion, is configured to become seated at the bladder neck and to locate the catheter in the delivery position.

### DISCLOSURE OF INVENTION

In light of the above circumstances, an object of the present invention is to provide a system comprising an endoscope and a balloon dilator for stably inflating a balloon at an appropriate position in a lumen of a living body, when it is used together with the endoscope, is further disclosed in claim 1

A system according to claim 1 is provided. The system may include an endoscope having a channel and a balloon dilator including:
a positioning part whose form is changed so as to be fit to the channel of the endoscope; and
a larger-diameter part, which is connected to the positioning part and is inflated to have a diameter larger than that of the positioning part.

In accordance with this balloon dilator, in an operation for dilating a narrowed or blocked part in a luminal organ by arranging the larger-diameter part in the luminal organ, while the larger-diameter part is inflated, or when the inflated larger-diameter part is moved forward or backward through the luminal organ, the positioning part is fit to the relevant channel by changing the form thereof, thereby stably positioning the larger-diameter part with respect to the endoscope.

In a preferable example, the positioning part is arranged on a side of the larger-diameter part, which is closer to an operator's hand, so that the positioning part communicates with and can be inflated together with the larger-diameter part.

In this case, the larger-diameter part can be inflated together with the positioning part while at least a part of the positioning part is contained in the channel. In this process, the positioning part can be fit to the channel.

In a typical example, when the form of the positioning part is changed, the positioning part has a substantially constant outer-diameter along the axial direction thereof.

In this case, when the form of the positioning part is changed so as to fit to the channel, any part of the positioning part can be fit to the channel. Therefore, the larger-diameter part can be positioned at a desired position with respect to the endoscope in a very stable manner.

In another typical example, when the form of the positioning part is changed, the outer diameter of the positioning part gradually decreases from the side connected to the larger-diameter part, to the other base end side.

In this case, when the form of the positioning part is changed, the positioning part can be fit to the channel at a specific position thereof between one end toward the larger-diameter part and the other end. Therefore, the balloon dilator can be used for a several kinds of diameters of endoscope channels.

In accordance with the present invention, when the larger-diameter part is inflated, it is possible to preferably prevent the larger-diameter part from being positioned at an erroneous spot, thereby reducing necessity of re-positioning and simplifying the operation.

### BRIEF DESCRIPTION OF THE DRAWINGS

Fig. 1 is a plan view showing a balloon dilator as a first embodiment in accordance with the present invention.
Fig. 2A is an enlarged view showing a main part of the balloon dilator in the first embodiment, and Fig. 2B is a front view showing the front end face of an endoscope, to which the balloon dilator of the first embodiment is inserted.
Fig. 3 is an enlarged view showing a main part of the balloon dilator in the first embodiment.
Fig. 4 is a diagram showing a method of using the balloon dilator of the first embodiment, and in particular, showing a state when the endoscope is inserted into a coelom.
Fig. 5A shows an example the endoscope image of a narrowed part, so as to explain the method of using the balloon dilator of the first embodiment, and Fig. 5B shows the endoscope in the vicinity of the narrowed part, also so as to explain the method of using the balloon dilator.
Fig. 6 shows a state in which the balloon dilator is inserted into a coelom via the endoscope, also so as to explain the method of using the balloon dilator.
Fig. 7 shows a state in which a guide wire is inserted to the narrowed part, also so as to explain the method of using the balloon dilator.
Fig. 8 shows a state in which the balloon is inflated and the shoulder part is fit to the channel, also so as to explain the method of using the balloon dilator.
Fig. 9A shows a state before the balloon dilator is contained in the channel, Fig. 9B shows a state in which the larger-diameter part is deflated, and Fig. 9C shows a state in which the balloon is inserted into the channel via the shoulder part, also so as to explain the method of using the balloon dilator.
Fig. 10 is an enlarged view showing a main part of another example of the balloon dilator in the first embodiment.
Fig. 11 is an enlarged view showing a main part of a balloon dilator as a second embodiment in accordance with the present invention.
Fig. 12 shows an example the endoscope image with respect to a state in which the balloon dilator of the second embodiment is protruded from the channel.
Fig. 13 is an enlarged view showing a main part of a balloon dilator as a third embodiment in accordance with the present invention.
Fig. 14 shows a state in which the balloon is inflated and the shoulder part is fit to the channel, so as to explain the method of using the balloon dilator of the third embodiment.

### BEST MODE FOR CARRYING OUT THE INVENTION

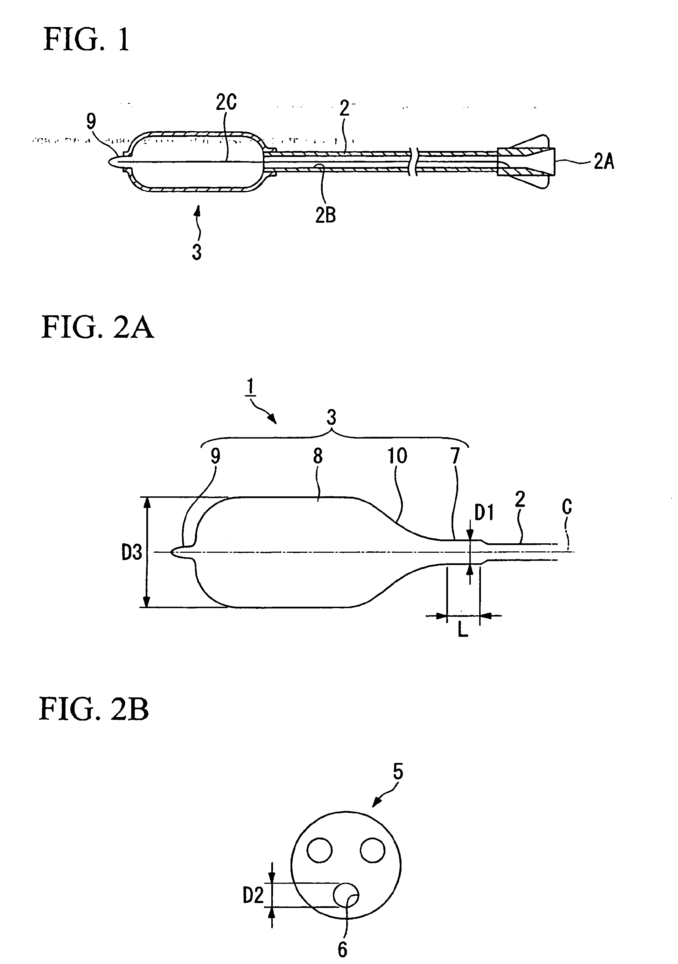

A first embodiment with respect to the present invention will be explained with reference to Figs. 1 to 9C.

As shown in Fig. 1, a balloon dilator 1 of the present embodiment has (i) a shaft 2, whose end has a sleeve 2A, and in which an inner opening 2B is arranged along the axial direction thereof, and (ii) a balloon 3 which is connected to and communicates with an end of the shaft 2.

As shown in Figs. 2A and 2B, the balloon 3 has (i) a shoulder part 7 (i.e., a positioning part) whose form can be changed so as to be fit to a channel 6 of an endoscope 5, (ii) a transit part 10 explained later, (iii) a larger-diameter part 8, which is connected to the shoulder part 7 via the transit part 10, and is inflated to have a diameter larger than that of the shoulder part 7, and (iv) a head part 9 arranged at the head of the larger-diameter part 8 so as to improve passing performance of the balloon 3.

When the balloon 3 is in a deflated state, it has a plurality of vanes, which protrude toward radial directions from the center axis C, and can be fold and wound around the center axis C.

The shaft 2 functions as a pipe passage for transmitting a liquid to the balloon 3 and absorbing it from the balloon 3. Through the inner opening 2B, a reinforcing wire 2C is inserted, whose head end is connected to the head part 9 of the balloon 3, and whose base end is connected to the sleeve 2A. The reinforcing wire 2C transmits a force for pushing the balloon 3 towards the head part 9.

The shoulder part 7 is arranged at the base side of the larger-diameter part 8, so that it can also be inflated while communicating with the larger-diameter part 8.

With respect to the outer diameter (D1) of the shoulder part 7 in the inflated state, for example, when the diameter (D2) of the channel 6 in the endoscope 5 is 2.8 mm, D1 is constant along the center axis C of the shoulder part 7. In addition, the length (L) of the axis of the shoulder part 7 is within the range of 5 to 15 mm.

The larger-diameter part 8 may be of a type which is generally called "semi-compliant", in which the diameter in the inflated state can be one of a few kinds of values in accordance with the amount and applied pressure of injected liquid. More specifically, the outer diameter (D3) in the inflated state is selectable within the range of 8 to 20 mm.

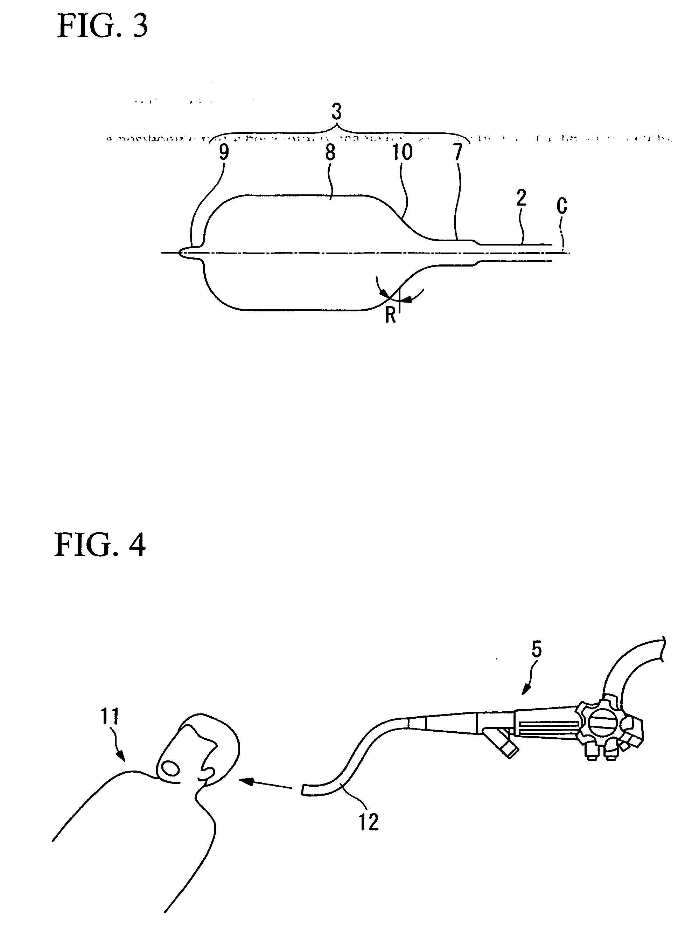

As shown in Fig. 3, when the larger-diameter part 8 and the shoulder part 7 are inflated, the transit part 10 is formed so as to couple the larger-diameter part to the shoulder part 7 at an inclination having an angle (R) of approximately 30 deg. with respect to the center axis C of the balloon 3.

Below, the method of usage, function, and effects of the balloon dilator 1 will be explained with regard to a case of applying the balloon dilator 1 to dilation using an endoscope for esophagus stenosis.

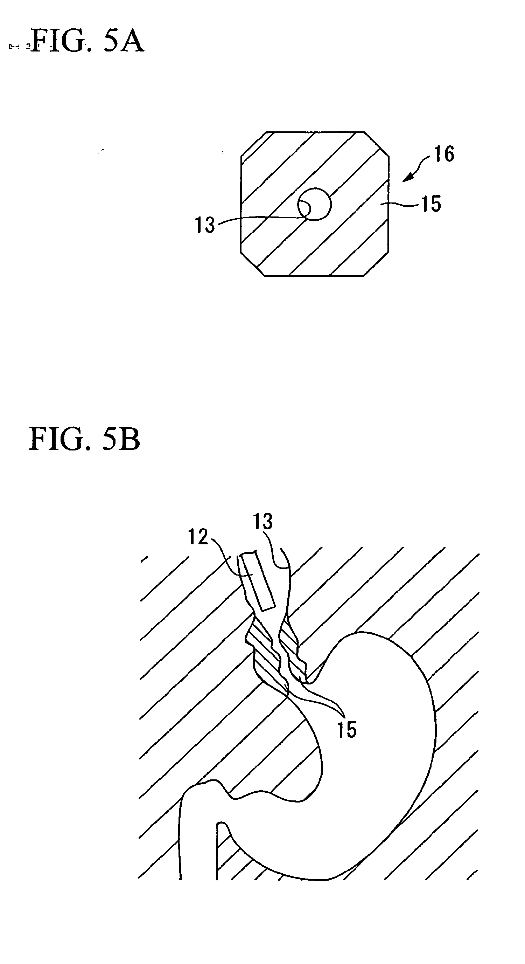

First, as shown in Fig. 4, an insertion part 12 of the endoscope 5 is inserted into the mouth of a patient 11, so as to confirm a narrowed part 15 of the esophagus 13 by means of an endoscope image 16 as shown in Fig. 5A, and the head of the insertion part 12 is positioned in the vicinity of the narrowed part 15 (see Fig. 5B).

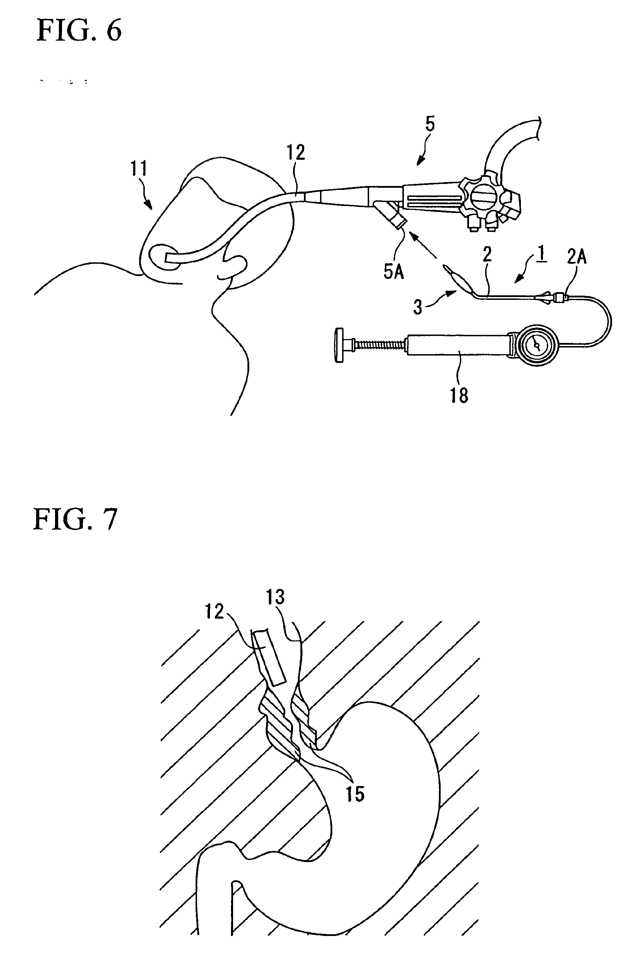

In this state, as shown in Figs. 6 and 7, the balloon dilator 1 is inserted into the channel 6 of the endoscope 5 from an insertion hole 5A thereof, wherein an inflation pump 18 is connected to the sleeve 2A.

Next, the larger-diameter part 8 of the balloon dilator 1 is protruded from the channel 6 so that the narrowed part 15 is positioned at the center of the larger-diameter part 8, and the shoulder part 7 is also protruded from the channel 6 in a manner such that at least a part of the shoulder part 7 remains in the channel 6.

Then, distilled water or the like is slowly injected by operating the inflation pump 18, so as to inflate both the larger-diameter part 8 and the shoulder part 7. In this process, first, the larger-diameter part 8 is inflated to have a predetermined minimum diameter for inflation, and the relevant pressure in this inflated state is maintained until the narrowed part 15 is appropriately dilated.

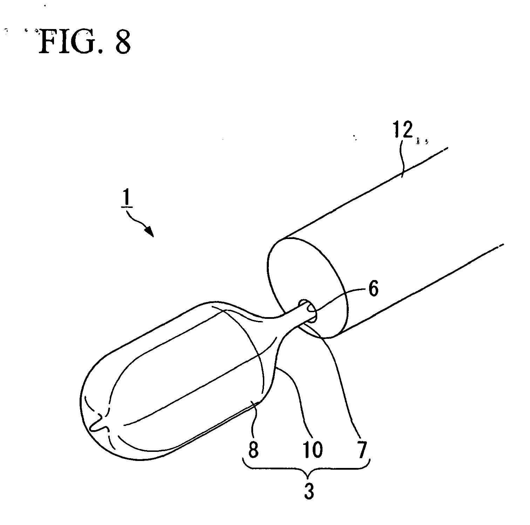

In this state, as shown in Fig. 8, the shoulder part 7 is inflated and fit to the channel 6, thereby restricting the movement of the balloon 3 with respect to the endoscope 5. Accordingly, the whole part of the balloon 3 is positioned with respect to the insertion part 12 of the endoscope 5. Therefore, it is possible to prevent the larger-diameter part 8 from being inflated at another position than the original target position of the narrowed part 15. Additionally, in order to further inflate the larger-diameter part 8, distilled water or the like is injected to provide a relevant specific pressure.

In Fig. 8 (and other relevant figures explained later), in order to show the relationship between the shoulder part 7 and the channel 6 in an easily-understandable manner, a gap is present between them at the exit of the insertion part 12 in the endoscope 5. However, in the state in which the shoulder part 7 is inflated and is fit to the channel 6 so as to restrict the axial movement, no gap is present between them, as shown by the above-described values of the outer diameters D1 and D2.

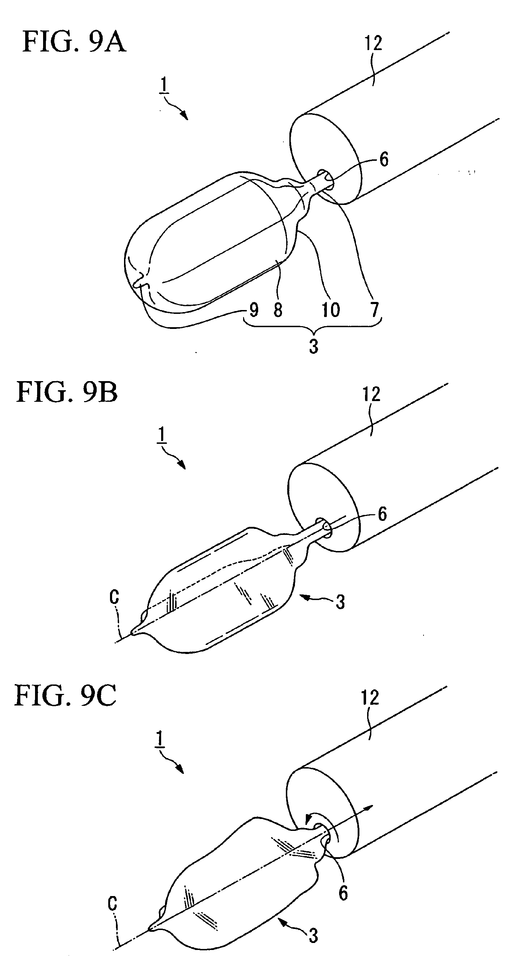

After the dilation, while the distilled water or the like in the balloon 3 is absorbed, the larger-diameter part 8 and the shoulder part 7 are deflated from a state shown in Fig. 9A, and their shape is changed to form vanes to be wound around the center axis C, thereby resulting in a deflated state as shown in Fig. 9B.

Therefore, the shoulder part 7 of the balloon 3, which is in a state as shown in Fig. 9C, is drawn into the channel 6, so that the balloon 3 is removed from the endoscope 5. If it is difficult to remove it, the winding-up operation may further be performed.

In accordance with the balloon dilator 1 having the shoulder part 7, when the larger-diameter part 8 is inflated, the shoulder part 7 is also inflated and can be fit to the channel 6 of the endoscope 5. In this process, as the shoulder part 7 has a constant diameter, any part of the shoulder part 7 can be fit to the channel 6. Therefore, the larger-diameter part 8 can be stably positioned and inflated at a target spot with respect to the endoscope 5 without slipping with respect to the narrowed part 15. Accordingly, positioning can be easily performed, thereby reducing the operation time.

In addition, when deflating the balloon 3 so as to contain it in the channel 6, the shoulder part 7 having a smaller diameter in comparison with the larger-diameter part 8 is first contained.

Therefore, the deflated larger-diameter part can be smoothly drawn into the channel 6.

Accordingly, it is possible to prevent an accident in which the balloon 3 cannot be smoothly contained in the channel 6, and thus to omit an extra operation for removing the balloon together with the endoscope. Therefore, the operation time can be reduced.

Furthermore, as the transit part 10 is provided, inflation can be performed while easily confirming the inner-peripheral face of the larger-diameter part 8 by means of the endoscope 5.

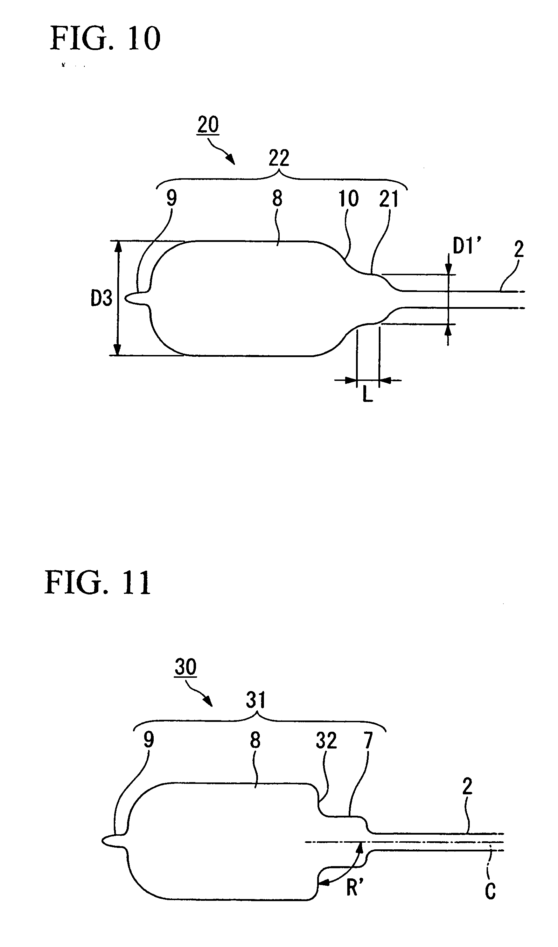

In another example, when the endoscope has a channel having a diameter of 3.7 mm, a balloon dilator 20 as shown in Fig. 10 may be used, which has a balloon 22 having a shoulder part 21, whose outer diameter D1' (in the inflated state) being within the range of 3.7 to 3.75 mm, thereby providing similar functions and effects.

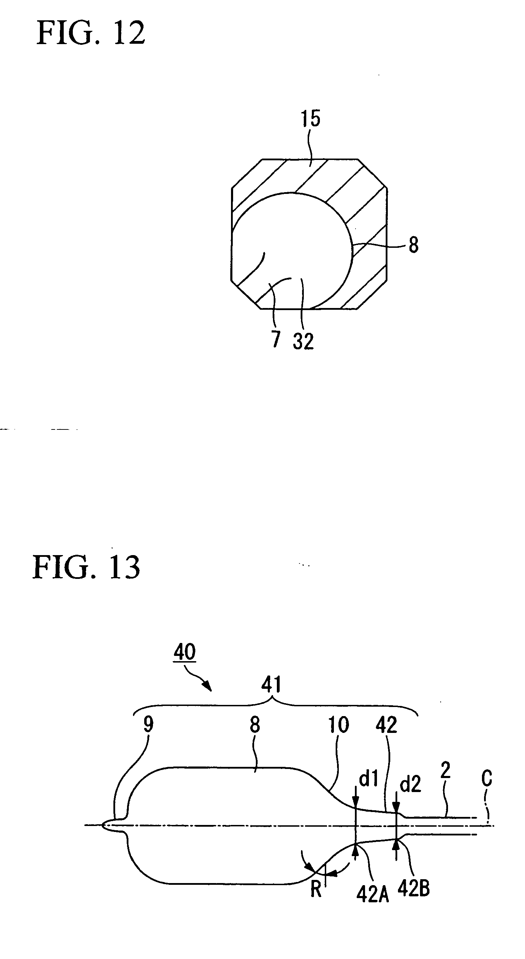

Below, a second embodiment will be explained with reference to Figs. 11 and 12.

Fig. 11 is an enlarged view showing a main part of a balloon dilator 30 in the second embodiment. Fig. 12 is a diagram showing an example of an endoscope-observed image in a state in which the balloon dilator 30 is protruded from a channel. Here, structural elements similar to those in the first embodiment are given identical reference numerals, and explanations thereof are omitted.

In comparison with the first embodiment, the second embodiment has a distinctive feature in which a balloon 31 of the balloon dilator 30 has a transit part 32, which rises from the shoulder part 7 at an angle R' of approximately 90° with respect to the center axis C of the balloon 31, so as to be connected to the larger-diameter part 8.

In accordance with the balloon dilator 30, similar functions and effects to those of the above-described first embodiment can be obtained, and the dilated state of a narrowed part can be observed by the endoscope from the inner side of the larger-diameter part 8 via the transit part 32.

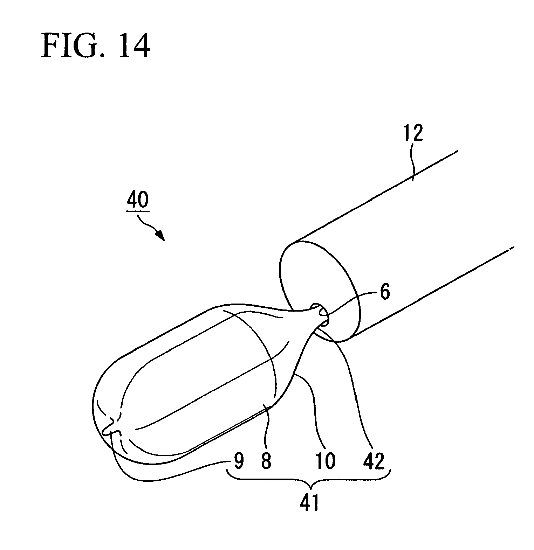

Below, a third embodiment will be explained with reference to Figs. 13 and 14.

Here, structural elements similar to those in the first embodiment are given identical reference numerals, and explanations thereof are omitted.

In comparison with the first embodiment, the third embodiment has a distinctive feature in which a balloon 41 of a balloon dilator 40 has a shoulder part 42, whose outer diameter generally decreases from the end closer to the larger-diameter part 8, to the end closer to the operator's hand.

As shown in Fig. 13, in the shoulder part 42 in the inflated state, one end part 42A, connected to the transit part 10, has an outer diameter d1 from 3.7 to 3.75 mm, while the other end part 42B, connected to the shaft 2, has an outer diameter d2 from 2.75 to 2.8 mm.

The length of the shoulder part 42 in the axial direction is within the range of 5 to 15 mm.

Therefore, a gentle taper part is formed between the end parts 42A and 42B of the shoulder part 42. As shown in Fig. 14, when the channel 6 of the endoscope 5 has a diameter of 2.8 mm, the shoulder part 42 is fit to the channel 6 at a specific position in the vicinity of the other end part 42B of the inflated shoulder part 42.

On the other hand, when the diameter of the channel is 3.7 mm, the shoulder part 42 is fit to the channel at a specific position in the vicinity of the one end part 42A of the inflated shoulder part 42.

In accordance with the balloon dilator 40, similar functions and effects to those of the first embodiment can be obtained.

In particular, as the shoulder part 42 has a tapered form, the shoulder part 42 can be fit to the channel 6 at a specific position along the center axis C. Therefore, the balloon dilator can be used for a several kinds of diameters of endoscope channels.

The technical range is not limited to the above-described embodiments, and various modifications are possible without departing from the scope of the present invention.

For example, in the embodiments, the shoulder part is a part of the balloon, however, it may be a separate part with respect to the balloon.

In addition, the shoulder part is pressured to be inflated, together with the larger-diameter part, by using distilled water or the like. However, it may be inflated and deformed by another method.

Furthermore, the balloon dilator can be applied, not only to a dilating operation using an endoscope, with respect to esophagus stenosis, but also to an operation applied to intestinal stenosis, or any narrowed or blocked part of a lumen in a living body.

## Claims

1. A system comprising:
an endoscope (5) comprising a channel (6); and
a balloon dilator having a balloon (3) comprising:
a shaft (2) having an inner opening arranged along an axial direction of the shaft (2),
**characterized in that** the balloon (3) includes:
a positioning part (7, 21, 42) which is connected to an end of the shaft (2) and is inflated to have a diameter larger than that of the shaft (2) so that a form of the positioning part (7, 21, 42) is changed so as to be fit to the channel (6) of the endoscope (5) in a manner such that at least a part of the positioning part (7, 21, 42) remains in the channel (6); and
a larger-diameter part (8), which is connected to the positioning part (7, 21, 42) via a transit part (10, 32) which is a part of the balloon (3) in a manner such that the positioning part (7, 21, 42) is interposed between the larger-diameter part (8) and the shaft (2), wherein the larger-diameter part (8) is inflated to have a diameter larger than that of the positioning part (7, 21, 42).

2. The system in accordance with claim 1, wherein the positioning part (7, 21, 42) is arranged on a side of the larger-diameter part (8), which is closer to an operator's hand, so that the positioning part (7, 21, 42) communicates with and can be inflated together with the larger-diameter part (8).

3. The system in accordance with any one of claims 1 and 2, wherein when the form of the positioning part (7, 21, 42) is changed, the positioning part (7, 21, 42) has a substantially constant outer-diameter along the axial direction thereof.

4. The system in accordance with any one of claims 1 and 2, wherein when the form of the positioning part (7) is changed, the outer diameter of the positioning part (7) gradually decreases from the side connected to the larger-diameter part (8), to the other base end side.

## Patentansprüche

1. System mit:
einem Endoskop (5) mit einem Kanal (6); und
einem Ballondilatator mit einem Ballon (3), der umfasst:
einen Schaft (2) mit einer entlang einer axialen Richtung des Schafts (2) angeordneten inneren Öffnung,
**dadurch gekennzeichnet, dass** der Ballon (3) aufweist:
ein Positionierungsteil (7, 21, 42), das mit einem Ende des Schafts (2) verbunden ist und zu einem Durchmesser aufgeblasen wird, der größer ist als der des Schafts (2), so dass sich eine Form des Positionierungsteils (7, 21, 42) so verändert, dass sie sich dem Kanal (6) des Endoskops (5) anpasst, in einer solchen Weise, dass zumindest ein Teil des Positionierungsteils (7, 21, 42) in dem Kanal (6) verbleibt; und
ein Teil mit größerem Durchmesser (8), das über ein einen Teil des Ballons (3) darstellendes Übergangsteil (10, 32) mit dem Positionierungsteil (7, 21, 42) so verbunden ist, dass das Positionierungsteil (7, 21, 42) zwischen dem Teil mit größerem Durchmesser (8) und dem Schaft (2) liegt, wobei das Teil mit größerem Durchmesser (8) zu einem Durchmesser aufgeblasen wird, der größer ist als der des Positionierungsteils (7, 21, 42).

2. System nach Anspruch 1, wobei das Positionierungsteil (7, 21, 42) an einer Seite des Teils mit größerem Durchmesser (8) angeordnet ist, die näher zur Hand einer Bedienungsperson liegt, so dass das Positionierungsteil (7, 21, 42) mit dem Teil mit größerem Durchmesser (8) in Verbindung steht und zusammen mit ihm aufgeblasen werden kann.

3. System nach einem der Ansprüche 1 und 2, wobei bei einer Veränderung der Form des Positionierungsteils (7, 21, 42), das Positionierungsteil (7, 21, 42) entlang seiner axialen Richtung einen im Wesentlichen gleichbleibenden Außendurchmesser aufweist.

4. System nach einem der Ansprüche 1 und 2, wobei bei einer Veränderung der Form des Positionierungsteils (7), der Außendurchmesser des Positionierungsteils (7) fortschreitend von der mit dem Teil mit größerem Durchmesser (8) verbundenen Seite zu der anderen Seite am Basisende abnimmt.

## Revendications

1. Système comprenant :
un endoscope (5) comprenant un canal (6) ; et
un dilatateur à ballonnet ayant un ballonnet (3) comprenant :
une tige (2) ayant une ouverture intérieure agencée le long d'un sens axial de la tige (2),
**caractérisé en ce que** le ballonnet (3) inclut :
une partie de positionnement (7, 21, 42) qui est raccordée à une extrémité de la tige (2) et est gonflée de manière à avoir un diamètre plus grand que celui de la tige (2), de telle sorte qu'une forme de la partie de positionnement (7, 21, 42) est changée de façon à être ajustée au canal (6) de l'endoscope (5) d'une manière telle qu'au moins une partie de la partie de positionnement (7, 21, 42) reste dans le canal (6) ; et
une partie de diamètre plus grand (8) qui est raccordée à la partie de positionnement (7, 21, 42) par l'intermédiaire d'une partie de transition (10, 32) qui est une partie du ballonnet (3) d'une manière telle que la partie de positionnement (7, 21, 42) est interposée entre la partie de diamètre plus grand (8) et la tige (2), dans lequel la partie de diamètre plus grand (8) est gonflée de façon à avoir un diamètre plus grand que celui de la partie de positionnement (7, 21, 42).

2. Système selon la revendication 1, dans lequel la partie de positionnement (7, 21, 42) est agencée sur un côté de la partie de diamètre plus grand (8), qui est plus proche d'une main d'un opérateur, de telle sorte que la partie de positionnement (7, 21, 42) communique avec et peut être gonflée en même temps que la partie de diamètre plus grand (8).

3. Système selon l'une quelconque des revendications 1 et 2, dans lequel, lorsque la forme de la partie de positionnement (7, 21, 42) est changée, la partie de positionnement (7, 21, 42) a un diamètre extérieur sensiblement constant le long du sens axial de celle-ci.

4. Système selon l'une quelconque des revendications 1 et 2, dans lequel, lorsque la forme de la partie de positionnement (7) est changée, le diamètre extérieur de la partie de positionnement (7) diminue graduellement du côté raccordé à la partie de diamètre plus grand (8) jusqu'à l'autre côté d'extrémité de base.
